# FASCICULE DE BREVET EUROPEEN

(11) **EP 0 740 557 B1**
(45) Date de publication et mention de la délivrance du brevet: **09.12.1998**
(21) Numéro de dépôt: 95907038.4
(22) Date de dépôt: 16.01.1995
(51) Int. Cl.: A61M 5/20

(54) **INJECTEUR AUTOMATIQUE DE MEDICAMENT**
AUTOMATISCHE MEDIKAMENTENEINSPRITZVORRICHTUNG
AUTOMATIC DRUG INJECTOR

(30) Priorité: 17.01.1994 FR 9400608
(43) Date de publication de la demande: 06.11.1996
(73) Titulaire: LABORATOIRE AGUETTANT, 69007 Lyon (FR)
(72) Inventeur: FREZZA, Pierre, F-69390 Vourles (FR)
(74) Mandataire: Maureau, Philippe
(86) Numéro de dépôt international: FR9500047
(87) Numéro de publication internationale: WO9519194

(56) Documents cités:
- EP-A- 0 516 473
- FR-A- 2 342 079
- FR-A- 2 654 938

## Description

L'invention concerne un injecteur automatique de médicament. Un tel injecteur permet de faire pénétrer à l'intérieur du corps d'un patient au travers de sa peau un médicament liquide. Pour ce faire, une aiguille creuse transperce la peau du patient, pénètre à une profondeur déterminée à l'intérieur du corps du patient, puis à l'aide d'un piston, le médicament liquide est injecté, à partir d'une ampoule ou du corps d'une seringue, à travers l'aiguille, dans le corps du patient.

Avec de tels injecteurs, une personne n'ayant pas l'habitude de faire des injections peut tout de même en faire. Il suffit pour cela de placer l'injecteur sur la peau du patient et de déclencher le mécanisme. Il est même possible de s'injecter soi-même un médicament, sans l'intervention d'un médecin ou d'un infirmier. Ainsi, il n'est plus nécessaire de prolonger l'hospitalisation d'une personne pour la seule raison qu'elle doit se faire injecter des médicaments plusieurs fois dans la journée.

Il est préférable que la personne qui reçoit le médicament ne voie pas l'aiguille, surtout si elle se fait elle-même l'injection. Il est ainsi plus facile pour elle de vaincre la peur de la piqûre.

Pour des raisons de sécurité, il est préférable que l'aiguille, après l'injection, soit protégée. Il est alors possible de simplement la protéger avec un capuchon, ou bien de la retirer de l'injecteur et de la placer dans un étui rigide. Mais il est avantageux que l'aiguille se rétracte automatiquement, sans intervention manuelle, afin d'obtenir une sécurité maximale. En effet, si le patient est porteur d'un virus, après l'injection, l'aiguille risque fort d'être contaminée, et si quelqu'un se blesse avec cette aiguille, la maladie (SIDA, Hépatite B, etc) véhiculée par le virus peut être transmise.

Le document WO 92/20388 révèle un injecteur automatique de ce type dans lequel l'aiguille ne se rétracte pas automatiquement en fin d'injection. Il y a donc risque de contamination avec un tel injecteur une fois qu'il a servi.

On connait également, par le document WO 92/18187, une seringue dans laquelle l'aiguille se rétracte automatiquement dans la tige du piston lorsque le piston est arrivé en fin de course. Cette seringue ne peut servir qu'une fois. D'autre part, seule la rétraction de l'aiguille est automatique. La piqûre et l'injection s'effectuent manuellement.

Le brevet FR 2 342 079 révèle quant à lui un injecteur dans lequel les trois opérations - piqûre, injection, rétraction - s'effectuent automatiquement. L'inconvénient de ce dispositif est qu'il faut, d'une part, libérer un premier ressort pour commander la piqûre et l'injection et, d'autre part, lorsque ces deux opérations sont effectuées, commander la rétraction de l'aiguille en libérant un autre ressort. Une fois l'injection faite, il est possible de retirer l'aiguille du corps du patient sans commander la rétraction de l'aiguille à l'intérieur du corps de l'injecteur. L'aiguille peut donc se trouver non protégée après l'injection et il y a alors risque de contamination.

Cet injecteur comprend notamment un corps cylindrique dans lequel coulisse axialement le corps d'une seringue, et trois ressorts. Un ressort commande l'introduction de l'aiguille dans le corps du patient, un autre l'injection du médicament et le troisième la rétraction de la seringue, avec l'aiguille, dans le corps cylindrique.

Le document EP-A-516473 décrit un injecteur selon le préambule de la revendication 1.

Le but de l'invention est de proposer un dispositif capable d'injecter une ou plusieurs doses de médicament, de manipulation très simple, permettant à un opérateur ne sachant pas faire des injections d'injecter une dose de médicament, éventuellement à lui-même, en préservant tout risque de piqûre accidentelle après utilisation du dispositif. Un autre but est de fournir un injecteur de fabrication simple et d'un prix de revient peu élevé.

A cet effet, l'injecteur qu'elle propose est un injecteur automatique de médicament sous forme liquide comprenant :
- un corps tubulaire destiné à être maintenu par l'utilisateur,
- un réservoir, à une ou plusieurs chambres, contenant le médicament à injecter comprenant une paroi tubulaire dans l'axe du corps, un piston formant joint d'étanchéité, fermant une extrémité de ladite paroi tubulaire, déplaçable vers l'autre extrémité où se trouve une aiguille creuse par laquelle s'effectue le passage du liquide, et comportant une tige de piston,
- deux ressorts comprimés emmagasinant l'énergie nécessaire pour le fonctionnement automatique de l'injecteur,
   caractérisé en ce que le premier ressort entoure au moins partiellement la tige du piston et est comprimé en prenant appui, d'une part, sur une partie de la surface intérieure du corps d'injecteur et, d'autre part, sur une pièce liée à la tige du piston du réservoir, ce premier ressort agissant dans un sens de sortie de l'aiguille, en ce que le second ressort qui agit en sens inverse du premier ressort, entoure au moins partiellement la paroi tubulaire du réservoir et est comprimé en prenant appui, d'une part, sur une partie de la surface intérieure du corps d'injecteur et, d'autre part, sur une bague montée coulissante le long de la surface extérieure du réservoir, susceptible d'entraîner le réservoir dans un sens de rétraction de l'aiguille, et pouvant être bloquée en translation par des moyens, comportant au moins une butée mobile, rappelée élastiquement vers l'intérieur du corps d'injecteur, en position de repos et contre laquelle la bague vient buter lorsqu'elle est en position de repos, en ce que la force exercée par le second ressort comprimé est supérieure à la force exercée par le premier ressort, et en ce que la pièce liée à la tige du piston du réservoir sur laquelle appuie le premier ressort coopère en fin de course du piston, avec les moyens de blocage de la bague pour libérer le second ressort et assurer la remontée du réservoir et la rétraction de l'aiguille à l'intérieur du corps de l'injecteur.

Ainsi, il suffit de libérer le premier ressort pour que successivement l'aiguille sorte du corps tubulaire et pénètre dans le corps du patient, que le liquide soit injecté par l'aiguille et que l'aiguille se rétracte automatiquement, puisque c'est la fin de la course d'injection qui commande la libération du second ressort.

De plus, très peu de pièces sont nécessaires pour la fabrication d'un tel injecteur : un réservoir, une aiguille, un piston avec sa tige de piston, une pièce liée à la tige de piston pouvant former avec celle-ci une pièce monobloc, deux ressorts, une bague, un corps tubulaire et des moyens de blocage de la bague. Pour assurer l'étanchéité et la stérilité de l'aiguille, au moins un capuchon est nécessaire, mais il n'a pas d'autre fonction technique.

Afin de limiter encore le nombre de pièces constituant l'injecteur et de diminuer de ce fait son prix de revient, les moyens de blocage de la bague seront avantageusement intégrés au corps tubulaire. Dans ce cas, les moyens de blocage de la bague comportent au moins une languette découpée dans le corps tubulaire ou rapportée à l'intérieur de celui-ci, reliée à ce dernier par son bord situé du côté opposé à l'aiguille et repliée vers l'intérieur du corps tubulaire.

Un tel injecteur peut être prévu pour injecter une seule dose de médicament, ou bien plusieurs.

Dans le premier cas, celui d'un injecteur unidose, étant donné le faible prix de revient du dispositif, celui-ci est jetable. Dans une forme d'exécution, la tige du piston comporte une zone de rétreint venant s'encliqueter dans le fond annulaire du corps tubulaire de l'injecteur, bloquant ainsi le piston en translation, et l'injection est déclenchée par la rupture de l'extrémité de la tige de piston dépassant du corps tubulaire.

Le fait de casser la tige du piston est une garantie d'inviolabilité de l'injecteur et prouve à l'utilisateur que l'injecteur qu'il utilise est neuf et n'a jamais servi.

Dans cette forme d'exécution, avantageusement, l'extrémité du réservoir située du côté opposé à l'aiguille comporte un rebord extérieur susceptible de passer librement entre les languettes du corps d'injecteur, sans modifier la position de celles-ci, et de prendre appui contre un épaulement ménagé à l'extrémité de la bague opposée à celle contre laquelle prend appui le second ressort, et la pièce liée à la tige de piston possède un diamètre au moins égal au diamètre extérieur de la bague, et est positionnée sur la tige de piston de telle sorte qu'en fin de course, elle soit disposée à proximité de la bague et ait écarté les languettes de blocage de cette dernière.

Si l'extrémité de l'aiguille est protégée, avant l'injection, par un capuchon transperçable monté en force sur le réservoir et comportant une paroi latérale très fine et déformable, il devient inutile de retirer ce capuchon avant de procéder à l'injection. Pour plus de garantie au niveau de l'étanchéité bactériologique, il est tout de même préférable de prévoir un second capuchon plus rigide, obturant l'ouverture inférieure du corps tubulaire par laquelle sort l'aiguille.

Dans le cas où l'injecteur peut injecter plusieurs doses de médicament, un doseur est combiné à l'injecteur. Dans ce doseur de type connu, la tige du piston comporte alors une pluralité de crans successifs et annulaires, régulièrement espacés selon l'axe du corps tubulaire, sur une longueur au moins égale à la course du piston nécessaire pour vider le réservoir, chaque cran ayant une section décroissante en se rapprochant du piston. La paroi tubulaire du réservoir, ainsi que la pièce liée à la tige du piston comportent au moins une dent élastique adaptée à une pénétration entre deux crans successifs de la tige de piston et coopérant avec ces crans. Selon l'invention, la pièce liée à la tige du piston permet le blocage en translation du piston vis-à-vis du corps tubulaire, par des moyens comportant au moins une butée sur le corps tubulaire contre laquelle vient en appui une partie mobile, rappelée élastiquement en position de repos, de la pièce liée à la tige du piston, et au moins une partie de la bague coulissant le long de la paroi extérieure du réservoir et sur laquelle le second ressort prend appui, fait saillie, à travers une lumière, hors du corps tubulaire de l'injecteur, permettant de ramener manuellement le second ressort détendu dans sa position initiale comprimée et bloquée.

Dans cette variante, les moyens de blocage de la pièce liée à la tige du piston comportent avantageusement au moins une première languette qui, formant la pièce liée à la tige du piston, est solidaire de cette dernière par son bord situé du côté opposé à l'aiguille et est inclinée de l'intérieur vers l'extérieur, de telle sorte qu'en position de repos, son bord situé du côté de l'aiguille vienne en appui contre une butée solidaire du corps tubulaire, et au moins une seconde languette découpée dans le corps tubulaire et reliée à ce dernier par son bord situé du côté opposé à l'aiguille fait face à une première languette de la pièce liée à la tige de piston, de façon à pouvoir faire échapper cette première languette de sa butée d'appui en appuyant sur la seconde languette du corps tubulaire. Comme précédemment, ceci permet de diminuer le nombre de pièces, de faciliter le montage du dispositif et de diminuer le prix de revient de l'ensemble.

Afin de limiter la course du piston et d'obtenir un dosage précis de la quantité de médicament injectée, la pièce liée à la tige du piston comporte au moins un ergot faisant saillie dans une fente axiale ménagée dans la paroi tubulaire du réservoir et dont la longueur est au moins égale à la longueur de l'ergot augmentée de la distance séparant deux crans de la tige du piston.

Dans cette forme d'exécution, avantageusement, en fin d'injection d'une dose, les languettes constitutives de la pièce liée à la tige du piston viennent en appui contre l'extrémité de la bague opposée à celle servant à l'appui du second ressort, et écartent les languettes de blocage de la bague.

De toute façon, l'invention sera bien comprise à l'aide de la description qui suit, en référence au dessin schématique annexé représentant, à titre d'exemples non limitatifs, deux formes d'exécution de ce dispositif.

Les figures 1 à 4 en représentent une première forme d'exécution.
Figure 1 est une vue en coupe longitudinale du dispositif en position de stockage,
Figure 2 en est une vue en coupe longitudinale lorsque l'aiguille pénètre dans le corps du patient,
Figure 3 en est une vue en coupe longitudinale à la fin de l'injection, avant la rétraction de l'aiguille,
Figure 4 en est une vue en coupe longitudinale après la rétraction de l'aiguille.
   Les figures 5 à 11 représentent une seconde forme d'exécution.

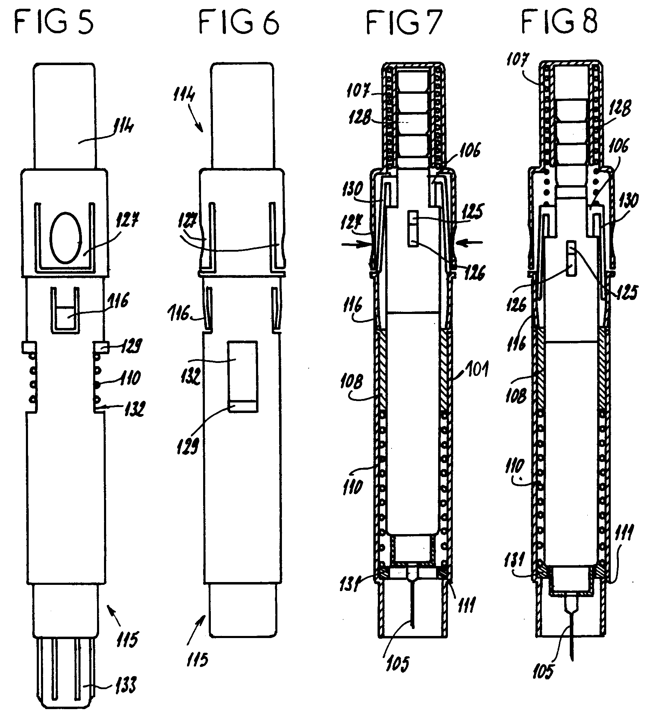

Figure 5 en est une vue de côté en position de stockage,
Figure 6 en est une vue d'un autre côté en position armée,
Figure 7 en est une vue en coupe longitudinale en position armée,
Figure 8 en est une vue en coupe longitudinale lorsque l'aiguille pénètre dans le corps du patient,

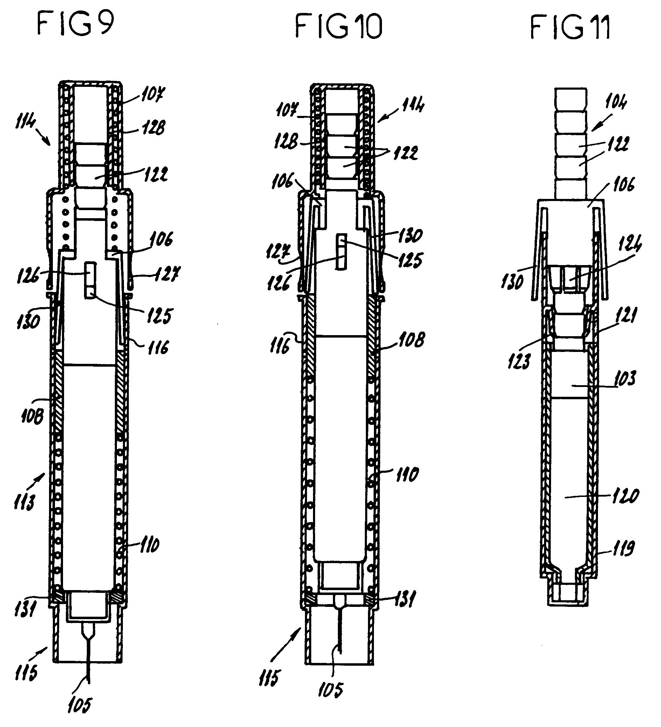

Figure 9 en est une vue en coupe longitudinale pendant l'injection,
Figure 10 en est une vue en coupe longitudinale en fin d'injection, lorsque l'aiguille est rétractée,
Figure 11 est une vue en coupe du réservoir de l'injecteur.

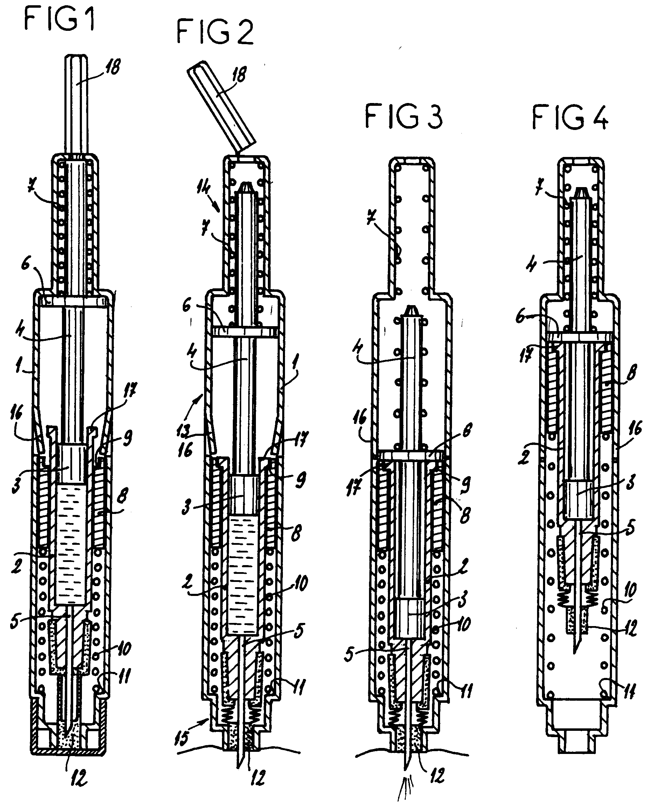

L'injecteur automatique de médicament représenté sur les figures 1 à 4 est un injecteur jetable destiné à ne faire qu'une seule injection.

Cet injecteur comporte :
- un corps tubulaire 1 destiné à être maintenu par l'utilisateur,
- une seringue comportant une paroi tubulaire 2 dans l'axe du corps 1 en verre ou en matière plastique, un piston 3 en élastomère pouvant coulisser dans la paroi tubulaire 2 formant le corps de la seringue, une tige de piston 4 et une aiguille 5 creuse,
- une collerette 6 liée à la tige du piston 4 et formant avec elle un ensemble monobloc,
- un premier ressort 7 entourant la tige de piston en position de stockage (Figure 1) s'appuyant, d'une part, sur le fond du corps tubulaire 1 et, d'autre part, sur la collerette 6,
- une bague 8, munie d'un épaulement 9, coulissant le long de la surface extérieure de la paroi tubulaire 2,
- un second ressort 10 entourant partiellement la paroi tubulaire 2 de la seringue, prenant appui, d'une part, sur la bague 8 et, d'autre part, sur un épaulement 11 du corps tubulaire 1,
- un capuchon 12 emmanché en force sur l'extrémité inférieure du corps de seringue, et
- un second capuchon rigide fermant l'extrémité inférieure du corps tubulaire 1.

Le corps tubulaire 1 est cylindrique. Il est constitué d'une partie centrale 13, d'une partie supérieure 14 et d'une partie inférieure 15. La partie supérieure 14 est d'un diamètre inférieur à celui de la partie centrale. La partie inférieure 15 est constituée de deux parties cylindriques de diamètres différents, reliées entre elles en formant un épaulement, et reliée à la partie centrale 13 en formant l'épaulement 11. Deux découpes en forme de U sont pratiquées dans la partie centrale 13, à une même altitude, en deux positions diamétralement opposées, la base du U étant orientée vers la partie inférieure 15 du corps tubulaire 1. Ces découpes forment deux languettes 16. Ces dernières sont repliées vers l'intérieur du corps tubulaire 1 et peuvent s'écarter élastiquement de leur position repliée.

A l'intérieur de ce corps tubulaire 1, se trouve une seringue. L'aiguille creuse 5 de cette seringue se situe du côté inférieur du corps 1. La paroi tubulaire 2 formant le corps de la seringue présente à son extrémité supérieure, celle opposée à l'aiguille 5, un rebord extérieur 17. Ce dernier peut passer librement entre les languettes 16 du corps d'injecteur 1, sans modifier la position de celles-ci.

Le médicament à injecter est contenu dans le corps de la seringue. Le côté supérieur du corps est fermé par le piston 3 en élastomère. L'autre côté est fermé par un fond sur lequel est fixée l'aiguille 5. Le capuchon 12 comporte un bouchon dans lequel est piquée l'extrémité de l'aiguille, assurant ainsi l'étanchéité au niveau de l'aiguille. Ce bouchon constitue le fond du capuchon cylindrique 12 emmanché en force sur l'extrémité inférieure du corps de la seringue. La paroi latérale de ce capuchon est très mince et déformable de façon à pouvoir se plier en accordéon lorsqu'elle est sollicitée à la compression. Ce capuchon 12 est par exemple en un élastomère, tel du bromobutyle. Le diamètre du bouchon constituant le fond du capuchon 12 est sensiblement égal au diamètre intérieur le plus faible de la partie inférieure 14 du corps 1.

La tige de piston 4 est reliée au piston 3, par vissage par exemple. Elle s'étend vers la partie supérieure 14 du corps tubulaire 1. L'extrémité 18 de la tige de piston 4, opposée au piston 3 est ruptible. La tige de piston 4 comporte une zone de rétreint constituant l'amorce d'une rupture. Cette zone se situe à la hauteur de l'extrémité supérieure du corps tubulaire 1. La partie supérieure 14 de ce corps 1 est munie d'un fond percé d'un trou de diamètre inférieur à celui de la tige de piston entre le piston et la zone de rétreint et supérieur au diamètre de la tige 4 dans la zone de rétreint. Ainsi, tant que l'extrémité de la tige n'est pas cassée, la tige de piston 4 est bloquée en translation par le fond annulaire du corps tubulaire 1.

La collerette 6 est solidaire de la tige de piston 4. Elle est située entre le piston 3 et la zone de rétreint, à une distance de la zone de rétreint supérieure ou égale à la longueur de la partie supérieure 14 du corps 1. Son diamètre est juste inférieur au diamètre intérieur de la partie centrale 13.

Le ressort 7 de diamètre intérieur supérieur à celui de la tige de piston 4 et de diamètre extérieur inférieur au diamètre intérieur de la partie supérieure 14 est comprimé entre la collerette 6 et le fond annulaire du corps 1 traversé par la tige 4.

La bague 8 est montée coulissante sur la paroi tubulaire 2 de la seringue. Son diamètre extérieur est inférieur ou égal à celui de la collerette 6. L'épaulement 9 est destiné à coopérer avec le rebord 17 de la paroi tubulaire 2.

La bague 8 est en butée contre les languettes 16. L'autre extrémité de la bague est en contact avec le ressort 10, qui prend appui, d'une part, sur la bague 8 et, d'autre part, sur l'épaulement 11 situé au niveau du raccordement de la partie centrale 13 du corps 1 et de sa partie inférieure 15.

Le second capuchon vient fermer la partie inférieure 15, afin de parfaire l'étanchéité bactériologique de l'injecteur.

Le fonctionnement de cet auto-injecteur à usage unique est le suivant.

Dans la position de stockage représentée sur la figure 1, les deux ressorts 7 et 10 sont comprimés, l'aiguille 5 est entièrement à l'intérieur du corps 1, son extrémité est protégée par le capuchon 12, l'extrémité inférieure de l'injecteur est recouverte par le second capuchon et la bague 8 vient en butée sous l'action du ressort 10 contre les languettes 16.

Lorsque l'on désire injecter le médicament contenu dans la seringue à un patient, il faut opérer comme suit. Le second capuchon est retiré. La partie inférieure 15 du corps 1 est alors posée sur la peau du patient, à l'endroit où il faut injecter le médicament. Il suffit alors de casser l'extrémité 18 de la tige du piston 4 (Figure 2) : la piqûre, l'injection et la rétraction de l'aiguille se font alors automatiquement.

En cassant l'extrémité 18 de la tige du piston 4, le ressort 7 comprimé est libéré. Prenant appui sur le fond du corps 1, il pousse la collerette 6. Cette force est alors transmise au piston 3. L'aiguille 5 étant bouchée et le médicament liquide étant incompressible, toute la seringue se déplace. Le bouchon du capuchon 12 est transpercé par l'aiguille 5 et les parois fines de ce capuchon 12 se plient en accordéon.

Lorsque le rebord 17 de la seringue vient en butée sur l'épaulement 9 de la bague 8, la descente de la seringue est stoppée. Le ressort 7 étant encore comprimé, il agit sur le piston 3 et injecte le médicament au patient.

Lorsque le piston 3 approche du fond du corps de seringue, la collerette 6 entre en contact avec les languettes 16, qui sont repliées vers l'intérieur du corps tubulaire 1 et empêchent la bague 8 de monter. En fin d'injection (Figure 3), les languettes 16 sont complètement écartées et ne servent plus de butée à la bague 8. La force exercée par le ressort 10 est nettement supérieure à la force exercée par le ressort 7. La bague 8 monte donc sous l'action du ressort 10, entraînant avec elle l'aiguille et toute la seringue, vidée de son contenu.

L'aiguille 5 se retire du corps du patient et se rétracte à l'intérieur du corps 1. Il est alors inutile, mais tout de même préférable, de remettre en place le second capuchon, car il n'y a aucun risque de blessure avec l'aiguille. L'injecteur peut alors être jeté, sans risque de contamination pour les tiers, puisque l'aiguille est parfaitement protégée par le corps 1.

Les figures 5 à 11 représentent une seconde forme d'exécution d'un injecteur selon l'invention. Dans cette forme d'exécution, tout le médicament contenu dans l'injecteur n'est pas injecté en une seule injection. Seule une dose de médicament est injectée par injection.

Dans cette seconde forme d'exécution, les éléments ayant la même fonction que dans la première forme d'exécution sont désignés par les mêmes références augmentées de 100.

Les différences les plus importantes entre les deux formes d'exécution sont le mode de déclenchement de l'injection et le remplacement de la seringue par un doseur.

La figure 11 représente un doseur en coupe longitudinale, sans le corps tubulaire 101 de l'injecteur.

Ce doseur comporte :
- un porte-carpule 119,
- une carpule en verre 120 pré-remplie logée dans le porte-carpule,
- un piston 103 fermant la carpule,
- un corps 121 prolongeant le porte-carpule 119,
- une tige de piston 104 reliée au piston 103, par vissage par exemple, et
- une pièce 106 montée sur la tige du piston 104.

La tige de piston 104 comporte une pluralité de crans 122 successifs et annulaires, régulièrement espacés selon l'axe du corps tubulaire, sur une longueur au moins égale à la course du piston nécessaire pour vider le réservoir. La hauteur d'un cran est égale à la course du piston nécessaire pour injecter une dose de médicament. Chaque cran a une section décroissante en se rapprochant du piston 103.

Le corps 121 est fixé sur le porte-carpule 119. Il a le même diamètre extérieur que celui-ci. Sur sa paroi intérieure, il comporte huit dents 123 élastiques adaptées par leur forme a une pénétration entre deux crans successifs 122 de la tige de piston 104. Lorsque les dents 123 sont entre deux crans 122, elles empêchent la tige de piston 104, et donc le piston 103, de coulisser dans le sens tendant à faire sortir le piston 103 de la carpule. Le piston 103 peut alors uniquement pousser le liquide.

La pièce 106 montée sur la tige du piston 104 comporte également huit dents élastiques 124 adaptées par leur forme à une pénétration entre deux crans successifs 122 de la tige de piston 104. Lorsque ces dents 124 sont entre deux crans successifs 122, celles-ci permettent un mouvement relatif entre la pièce 106 et le piston 103, uniquement dans le sens où ces deux pièces s'éloignent l'une de l'autre. D'autre part, si l'on exerce une force sur la pièce 106 en direction du piston 103 lorsque les dents sont entre deux crans successifs, cette force est entièrement transmise par l'intermédiaire de la tige 104 au piston 103.

La pièce 106 comporte également deux ergots 125 faisant saillie à l'intérieur d'une lumière 126 ménagée dans le corps 121. Ces lumières 126 ont une longueur légèrement supérieure à la longueur des ergots 125 augmentée de la distance séparant deux crans 122 de la tige de piston 104, pour tenir compte du jeu de fonctionnement du doseur.

La pièce 106 comporte également deux languettes 130 élastiques, se prolongeant le long du corps 121, à l'extérieur de celui-ci, et légèrement inclinées par rapport à l'axe du doseur.

Un tel doseur est monté dans un corps tubulaire 101. Comme le corps tubulaire 1 décrit ci-dessus, le corps tubulaire 101 comporte une partie centrale 113, une partie supérieure 114 de diamètre inférieur à celui de la partie centrale 113, une partie inférieure 115, deux languettes 116 et un épaulement 111.

Deux languettes 127 supplémentaires sont ménagées dans le corps 101 proprement dit. Une découpe en forme de U, (comme le montre la figure 5), la base du U étant orientée vers la partie inférieure du corps 101, à deux endroits diamétralement opposés, forme ces languettes 127, qui ne sont pas repliées vers l'intérieur du corps 101, contrairement aux languettes 116.

Une paroi 128 ménagée dans la partie supérieure 114 du corps 101 est destinée à séparer la tige du piston 104 d'un ressort 107.

Deux lumières 132 sont prévues également dans le corps 101 pour laisser passer deux ergots 129 d'une bague 108. La bague 108 correspond à la bague 8 de la première variante décrite. Elle diffère de la bague 8 en ce qu'elle présente deux ergots 129 diamétralement opposés et en ce que l'épaulement 9 est devenu superflu.

Une butée élastique 131 est montée au niveau de l'épaulement 111 existant entre le corps 101 proprement dit et sa partie inférieure 115. Un ressort 110 est comprimé entre cette butée 131 et la bague 108. Le ressort 107 est quant à lui comprimé entre le fond de la partie supérieure 114 - qui n'est pas pourvu d'une ouverture - et la pièce 106.

Une aiguille 105 est montée sur la partie inférieure du porte-capsule 119. Un capuchon 133 protège celle-ci.

Le fonctionnement de cet injecteur est le suivant.

En position de stockage (Figure 5), le ressort 107 est comprimé, le ressort 110 est détendu, la bague 108 est en position haute, les ergots 125 de la pièce 106 liée à la tige du piston 104 sont en butée dans la partie supérieure des lumières 126 et les languettes 127 du corps 101 font face aux languettes 130 de la pièce 106.

Avant de se servir de l'injecteur, il faut l'armer. Grâce aux deux ergots 129 de la bague 108 guidés dans les lumières 132, la bague 108 est descendue en direction de l'aiguille 105. Elle comprime alors le ressort 110. Lorsque la partie supérieure de la bague 108 est en dessous des languettes 116, celles-ci se replient vers l'intérieur du corps tubulaire 101, dans leur position de repos, et empêchent la bague 108 de remonter, malgré la poussée du ressort 110. Les languettes 130 de la pièce 106 liée à la tige de piston 104 qui reposaient sur la bague 108, descendent sous l'action du ressort 107. Mais elles sont arrêtées au niveau des découpes en forme de U réalisées pour former les languettes 127 et qui forment une butée (Figures 6 et 7).

Il suffit alors de retirer le capuchon 133 et le dispositif est prêt à fonctionner.

La partie avant de l'injecteur est alors posée sur la zône à piquer. Pour déclencher la piqûre, l'injection et la rétraction du doseur, il suffit d'appuyer sur les deux languettes flexibles 127 (Figure 7). Ces dernières transmettent alors le mouvement aux languettes 130 qui échappent de ce fait à la butée formée par les découpes en forme de U. Le ressort 107 est alors libéré et exerce une force vers le bas sur la pièce 106. Les dents 121 étant entre deux crans successifs de la tige de piston 104, cette force est transmise par l'intermédiaire du piston 103 et du liquide incompressible à injecter sur l'ensemble du doseur. L'aiguille 105 sort alors du dispositif jusqu'à ce que la partie inférieure du porte-carpule 119 vienne en butée sur la butée élastique 131 (Figure 8). Le ressort 107 continue alors d'exercer une force sur la pièce 106. Cette force permet l'injection d'une dose de médicament. Les ergots 125 se déplacent dans les lumières 126. Pendant l'injection (Figure 8), et surtout peu avant la fin de celle-ci, les languettes 130 de la pièce 106 écartent les languettes 116 du corps tubulaire 101. Au moment où les ergots 125 arrivent en butée dans les lumières 126, les languettes 116 sont suffisamment écartées pour libérer la bague 108 qui est poussée par le ressort 110. La force exercée par ce dernier est supérieure à celle exercée par le ressort 107. La bague 108 par l'intermédiaire des languettes 130 pousse donc la pièce 106 vers le haut. La force exercée par le ressort 110 est suffisante pour remonter l'ensemble doseur et faire coulisser la pièce 106 d'un cran 122 sur la tige de piston 104. Les ergots 125 se retrouvent alors en butée dans la partie supérieure des fentes 126.

Il suffit alors de remettre le capuchon 133 en place. L'injecteur est en position de stockage. Même si l'on omet de remettre le capuchon 133, l'aiguille 105 est protégée à l'intérieur du corps 101 et il n'y a aucun risque de se blesser avec elle. En effet, le ressort 110 étant libéré et exerçant une force supérieure à celle du ressort 107, même une action intempestive sur les languettes 127 sera sans effet sur le doseur et l'aiguille qui resteront protégés dans le corps 101.

Comme il va de soi, l'invention ne se limite pas aux formes d'exécution décrites ci-dessus à titre d'exemple, elle en embrasse au contraire toutes les variantes.

Ainsi par exemple, dans une autre variante non décrite, la seringue ou le doseur peuvent être remplacés par une seringue à plusieurs chambres. De façon connue, les divers produits contenus dans les chambres sont alors mélangés avant de procéder à l'injection.

## Revendications

1. Injecteur automatique de médicament sous forme liquide comprenant :
- un corps tubulaire (1,101) destiné à être maintenu par l'utilisateur,
- un réservoir, à une ou plusieurs chambres, contenant le médicament à injecter comprenant une paroi tubulaire (2,102) dans l'axe du corps, un piston (3,103) formant joint d'étanchéité, fermant une extrémité de ladite paroi tubulaire (2,102), déplaçable vers l'autre extrémité où se trouve une aiguille creuse (5,105) par laquelle s'effectue le passage du liquide, et comportant une tige de piston (4,104),
- deux ressorts (7,10,107,110) comprimés emmagasinant l'énergie nécessaire pour le fonctionnement automatique de l'injecteur, le premier ressort (7,107) agissant dans un sens de sortie de l'aiguille (5,105), et le second ressort (10,110) agissant en sens inverse du premier ressort (7,107), entourant au moins partiellement la paroi tubulaire (2,102) du réservoir et étant comprimé en prenant appui sur une partie de la surface intérieure du corps (1,101) d'injecteur
caractérisé en ce que le premier ressort (7,107) entoure au moins partiellement la tige du piston (4,104) et est comprimé en prenant appui, d'une part, sur une partie de la surface intérieure du corps d'injecteur et, d'autre part, sur une pièce (6,106) liée à la tige du piston (4,104) du réservoir, en ce que le second ressort prend appui sur une bague (8,108) montée coulissante le long de la surface extérieure du réservoir, susceptible d'entraîner le réservoir dans un sens de rétraction de l'aiguille (5,105), et pouvant être bloquée en translation par des moyens, comportant au moins une butée mobile (16,116), rappelée élastiquement vers l'intérieur du corps d'injecteur, en position de repos et contre laquelle la bague (8,108) vient buter lorsqu'elle est en position de repos, en ce que la force exercée par le second ressort (10,110) comprimé est supérieure à la force exercée par le premier ressort (7,107), et en ce que la pièce (6,106) liée à la tige du piston du réservoir sur laquelle appuie le premier ressort (7,107) coopère en fin de course du piston, avec les moyens de blocage de la bague (8,108) pour libérer le second ressort (10,110) et assurer la remontée du réservoir et la rétraction de l'aiguille à l'intérieur du corps (1,101) de l'injecteur.

2. Injecteur automatique selon la revendication 1, caractérisé en ce que les moyens de blocage de la bague (8,108) comportent au moins une languette (16,116) découpée dans le corps tubulaire (1,101) ou rapportée à l'intérieur de celui-ci, reliée à ce dernier par son bord situé du côté opposé à l'aiguille (5,105) et repliée vers l'intérieur du corps tubulaire (1,101).

3. Injecteur automatique selon l'une quelconque des revendications 1 et 2, caractérisé en ce que, dans le cas d'un injecteur à usage unique, la tige du piston (4) comporte une zone de rétreint venant s'encliqueter dans le fond annulaire du corps tubulaire (1) de l'injecteur, bloquant ainsi le piston (3) en translation, et en ce que l'injection est déclenchée par la rupture de l'extrémité de la tige de piston (18) dépassant du corps tubulaire (1).

4. Injecteur automatique selon la revendication 3, caractérisé en ce que l'extrémité du réservoir située du côté opposé à l'aiguille (5) comporte un rebord extérieur (17) susceptible de passer librement entre les languettes (16) du corps d'injecteur (1), sans modifier la position de celles-ci, et de prendre appui contre un épaulement (9) ménagé à l'extrémité de la bague (8) opposée à celle contre laquelle prend appui le second ressort (10), et en ce que la pièce (6) liée à la tige de piston (4) possède un diamètre au moins égal au diamètre extérieur de la bague (8), et est positionnée sur la tige de piston (4) de telle sorte qu'en fin de course, elle soit disposée à proximité de la bague (8) et ait écarté les languettes (16) de blocage de cette dernière.

5. Injecteur automatique selon l'une des revendications 3 et 4, caractérisé en ce que l'extrémité de l'aiguille (5) est protégée, avant l'injection, par un capuchon transperçable (12) monté en force sur le réservoir et comportant une paroi latérale très fine et déformable.

6. Injecteur automatique selon l'une quelconque des revendications 1 et 2, dont la tige de piston (104) comporte une pluralité de crans (122) successifs et annulaires, régulièrement espacés selon l'axe du corps tubulaire, sur une longueur au moins égale à la course du piston nécessaire pour vider le réservoir, chaque cran (122) ayant une section décroissante en se rapprochant du piston (103), injecteur dont la paroi tubulaire (102) du réservoir comporte au moins une dent élastique (123) adaptée à une pénétration entre deux crans successifs (122) de la tige de piston (104) et coopérant avec ces crans (122), et dont la pièce (106) liée au piston comporte au moins une dent élastique (124) adaptée à une pénétration entre deux crans successifs (122) de la tige de piston (104) et coopérant avec ces crans (122), caractérisé en ce que la pièce (106) liée à la tige du piston (104) permet le blocage en translation du piston vis-à-vis du corps tubulaire, par des moyens comportant au moins une butée sur le corps tubulaire contre laquelle vient en appui une partie mobile (130), rappelée élastiquement en position de repos, de la pièce (106) liée à la tige du piston (104), et en ce qu'au moins une partie (129) de la bague coulissant le long de la paroi extérieure du réservoir et sur laquelle le second ressort (110) prend appui, fait saillie, à travers une lumière (132), hors du corps tubulaire de l'injecteur (101), permettant de ramener manuellement le second ressort (110) détendu dans sa position initiale comprimée et bloquée.

7. Injecteur automatique selon la revendication 6, caractérisé en ce que les moyens de blocage de la pièce (106) liée à la tige du piston (104) comportent au moins une première languette (130) qui, formant la pièce liée à la tige du piston, est solidaire de cette dernière par son bord situé du côté opposé à l'aiguille et est inclinée de l'intérieur vers l'extérieur, de telle sorte qu'en position de repos, son bord situé du côté de l'aiguille vienne en appui contre une butée solidaire du corps tubulaire, et en ce qu'au moins une seconde languette (127) découpée dans le corps tubulaire (101) et reliée à ce dernier par son bord situé du côté opposé à l'aiguille fait face à une première languette (130) de la pièce liée à la tige de piston (104), de façon à pouvoir faire échapper cette première languette (130) de sa butée d'appui en appuyant sur la seconde languette (127) du corps tubulaire (101).

8. Injecteur automatique selon l'une des revendications 6 et 7, caractérisé en ce que la pièce (106) liée à la tige du piston (104) comporte au moins un ergot (125) faisant saillie dans une fente axiale (126) ménagée dans la paroi tubulaire du réservoir et dont la longueur est au moins égale à la longueur de l'ergot (125) augmentée de la distance séparant deux crans (122) de la tige du piston (104).

9. Injecteur automatique selon l'une des revendications 6 à 8, caractérisé en ce qu'en fin d'injection d'une dose, les languettes (130) constitutives de la pièce liée à la tige du piston viennent en appui contre l'extrémité de la bague opposée à celle servant à l'appui du second ressort, et écartent les languettes (116) de blocage de la bague (108).

## Claims

1. Automatic injector for a drug in liquid form comprising:
- a tubular body (1, 101) intended to be held by the user;
- a reservoir with one or more chambers containing the drug to be injected, comprising a tubular wall (2, 102) in the axis of the body, a piston (3, 103) forming a sealed joint, enclosing one end of the said tubular wall (2, 102) and displaceable towards the other end where there is a hollow needle (5, 105) through which the liquid is expelled, and comprising a piston rod (4, 104);
- two compressed springs (7, 10, 107, 110) storing the energy necessary for automatic operation of the injector, the first spring (7, 107) acting in the direction of outlet of the needle (5, 105) and the second spring (10, 110) acting in the opposite direction to the first spring (7, 107), surrounding at least partly the tubular wall (2, 102) of the reservoir and being compressed by resting on a part of the inner surface of the body (1, 101) of the injector;
characterised in that the first spring (7, 107) surrounds at least partly the piston rod (4, 104) and is compressed by being supported firstly on part of the inner surface of the injector body and secondly on a piece (6, 106) linked to the piston rod (4, 104) of the reservoir, in that the second spring rests on a ring (8, 108) mounted sliding along the outer surface of the reservoir, able to guide the reservoir in the direction of retraction of the needle (5, 105) and which can be blocked in translation by means comprising at least one mobile stop (6, 116) returned elastically towards the inside of the injector body in the rest position and against which the ring (8, 108) stops when the said ring is in the rest position, in that the force exerted by the second compressed spring (10, 110) is greater than the force exerted by the first spring (7, 107), and in that the piece (6, 106) linked to the piston rod of the reservoir on which rests the first spring (7, 107) co-operates at the end of travel of the piston with the means (8, 108) for blocking the ring in order to release the second spring (10, 110) and ensure the return of the reservoir and the retraction of the needle inside the body (1, 101) of the injector.

2. Automatic injector according to claim 1, characterised in that the means (8, 108) for blocking the ring comprise at least one tab (16, 116) cut out of the tubular body (1, 101) or built up inside this, linked to the latter by its edge located at the side opposite the needle (5, 105) and folded towards the inside of the tubular body (1, 101).

3. Automatic injector according to any of claims 1 and 2, characterised in that in the case of a single use injector, the piston rod (4) comprises a contracted zone which engages in the annular base of the tubular body (1) of the injector, thus blocking the movement of the piston (3), and in that the injection is triggered by the breakage of the end of the piston rod (18) projecting from the tubular body (1).

4. Automatic injector according to claim 3, characterised in that the end of the reservoir situated at the side opposite the needle (5) comprises an outer shoulder (17) able to pass freely between the tabs (16) of the injector body (1) without modifying their position, and to rest against a collar (9) arranged at the end of the ring (8) opposite that against which rests the second spring (10), and in that the piece (6) linked to the piston rod (4) has a diameter at least equal to the outer diameter of the ring (8) and is positioned on the piston rod (4) such that at the end of travel it is positioned close to the ring (8) and has parted the tabs (16) blocking the latter.

5. Automatic injector according to any of claims 3 and 4, characterised in that the end of the needle (5) is protected before injection with a pierceable cover (12) force-mounted on the reservoir and comprising a very fine and deformable side wall.

6. Automatic injector according to any of claims 1 and 2, of which the piston rod (104) comprises a plurality of successive annular notches (122) regularly spaced according to the axis of the tubular body, over a length at least equal to the travel of the piston necessary to evacuate the reservoir, each notch (122) having a decreasing section in the direction towards the piston (103), an injector of which the tubular wall (102) of the reservoir comprises at least one elastic tooth (123) able to penetrate between two successive notches (122) of the piston rod (104) and cooperating with these notches (122), of which the piece (106) linked to the piston has at least one elastic tooth (124) able to penetrate between two successive notches (122) of the piston rod (104) and co-operating with these notches (122), characterised in that the piece (106) linked to the piston rod (104) allows the movement of the piston in relation to the tubular body to be blocked by means comprising at least one stop on the tubular body against which rests a mobile part (130), returned elastically to a rest position, of the piece (106) linked to the piston rod (104), and in that at least one part (129) of the ring sliding along the outer wall of the reservoir and on which rests the second spring (110) projects through a slot (132) outside the tubular body of the injector (101) allowing manual return of the second extended spring (110) into its initial compressed and blocked position.

7. Automatic injector according to claim 6, characterised in that the means of blocking the piece (106) linked to the piston rod (104) comprise at least one first tab (130) which, forming the piece linked to the piston rod, is joined to the latter by its edge situated at the side opposite the needle and is angled from the inside towards the outside such that in its rest position, its edge situated at the needle side rests against a stop attached to the tubular body, and in that at least one second tab (127) cut out of the tubular body (101) and linked to the latter by its edge situated at the side opposite the needle faces a first tab (130) of the piece linked to the piston rod (104) so as to be able to move the first tab (130) from its stop while resting on the second tab (127) of the tubular body (101).

8. Automatic injector according to any of claims 6 and 7, characterised in that the piece (106) linked to the piston rod (104) comprises at least one lug (125) projecting into an axial slot (126) arranged in the tubular wall of the reservoir, and of which the length is equal at least to the length of the lug (125) plus the distance separating the two notches (122) of the piston rod (104).

9. Automatic injector according to any of claims 6 to 8, characterised in that on completion of the injection of a dose, the tabs (130) constituting the piece linked to the piston rod come to rest against the end of the ring opposite that serving as support for the second spring, and part the tabs (116) blocking the ring (108).

## Patentansprüche

1. Automatikspritze für Flüssigarzneien, umfassend:
- einen Rohrkörper (1, 101), der dazu bestimmt ist, vom Benutzer gehalten zu werden,
- ein die zu spritzende Arznei enthaltendes Ein- oder Mehrkammer-Reservoir mit einer Rohrwand (2, 102) in Achsrichtung des Körpers, wobei ein eine Dichtung bildender Kolben (3, 103) ein Ende dieser Rohrwand (2, 102) verschließt, zum anderen Ende hin verlagerbar ist, wo sich eine Hohlnadel (5, 105) befindet, durch die der Durchgang der Flüssigkeit erfolgt, und eine Kolbenstange (4, 104) aufweist,
- zwei komprimierte Federn (7, 10, 107, 110), welche die zum Automatikbetrieb der Spritze notwendige Energie speichern, wobei die erste Feder (7, 107) in Richtung der Ausmündung der Nadel (5, 105) wirkt und wobei die zweite Feder (10, 110) in umgekehrter Richtung zur ersten Feder (7, 107) wirkt, die Rohrwand (2, 102) des Reservoirs zumindest teilweise umschließt und unter Abstützung an einem Teil der Innenseite des Spritzenkörpers (1, 101) komprimiert ist,
**dadurch gekennzeichnet**, daß die erste Feder (7, 107) die Kolbenstange (4, 104) zumindest teilweise umschließt und unter Abstützung einerseits an einem Teil der Innenseite des Spritzenkörpers und andererseits an einem mit der Kolbenstange (4, 104) des Reservoirs verbundenen Stück (6, 106) komprimiert ist, daß die zweite Feder an einem längs der Außenseite des Reservoirs verschiebbar angeordneten Ringelement (8, 108) abgestützt ist, welches das Reservoir in einer Rückziehrichtung der Nadel (5, 105) mitzunehmen vermag und gegen Längsbewegung durch Mittel blockiert werden kann, welche mindestens einen zum Innern des Spritzenkörpers hin elastisch in eine Ruheposition rückgestellten, beweglichen Anschlag (16, 116) umfassen, gegen den das Ringelement (8, 108) anstößt, wenn dieser sich in der Ruhestellung befindet, daß die von der komprimierten zweiten Feder (10, 110) ausgeübte Kraft größer als die von der ersten Feder (7, 107) ausgeübte Kraft ist und daß das mit der Kolbenstange des Reservoirs verbundene Stück (6, 106), an dem die erste Feder (7, 107) abgestützt ist, am Ende des Kolbenhubs mit den Mitteln zum Blockieren des Ringelements (8, 108) zusammenwirkt, um die zweite Feder (10, 110) freizugeben und den Einzug des Reservoirs und den Rückzug der Nadel in das Innere des Körpers (1, 101) der Spritze zu bewirken.

2. Automatikspritze nach Anspruch 1,
**dadurch gekennzeichnet**, daß die Mittel zum Blockieren des Ringelements (8, 108) mindestens eine aus dem Rohrkörper (1, 101) ausgeschnittene oder innen an diesem angebrachte Zunge (16, 116) umfassen, welche mit ihrem auf der gegenüberliegenden Seite der Nadel (5, 105) liegenden Rand mit dem Rohrkörper (1, 101) verbunden ist und zum Innern des Rohrkörpers (1, 101) hin gebogen ist.

3. Automatikspritze nach einem der Ansprüche 1 und 2,
**dadurch gekennzeichnet**, daß im Fall einer Spritze zum Einmalgebrauch die Kolbenstange (4) eine Einschnürzone aufweist, welche in den ringförmigen Boden des Rohrkörpers (1) der Spritze einrastet und so den Kolben (3) gegen Längsbewegung blockiert, und daß die Injektion durch Abbrechen des aus dem Rohrkörper (1) herausragenden Endes der Kolbenstange (18) ausgelöst wird.

4. Automatikspritze nach Anspruch 3,
**dadurch gekennzeichnet**, daß das auf der gegenüberliegenden Seite der Nadel (5) liegende Ende des Reservoirs einen Außenrand (17) aufweist, welcher frei zwischen den Zungen (16) des Spritzenkörpers (1) hindurchgehen kann, ohne deren Stellung zu verändern, und zur Anlage an einer Schulter (9) gelangen kann, welche an demjenigen Ende des Ringelements (8) ausgebildet ist, das dem gegenüber liegt, an dem die zweite Feder (10) abgestützt ist, und daß das mit der Kolbenstange (4) verbundene Stück (6) einen Durchmesser besitzt, der mindestens gleich dem Außendurchmesser des Ringelements (8) ist, und derart auf der Kolbenstange (4) positioniert ist, daß es sich am Hubende nahe des Ringelements (8) befindet und die Blockierzungen (16) des letzteren auseinandergespreizt hat.

5. Automatikspritze nach einem der Ansprüche 3 und 4,
**dadurch gekennzeichnet**, daß das Ende der Nadel (5) vor der Injektion durch eine durchbohrbare Verschlußkappe (12) geschützt ist, welche fest an dem Reservoir angebracht ist und eine sehr dünne und verformbare Seitenwand besitzt.

6. Automatikspritze nach einem der Ansprüche 1 und 2, deren Kolbenstange (104) eine Mehrzahl aufeinanderfolgender und ringförmiger Raststufen (122) aufweist, welche mit gleichmäßigen Abständen längs der Achse des Rohrkörpers auf einer Länge angeordnet sind, die mindestens gleich dem zum Leeren des Reservoirs notwendigen Kolbenhub ist, wobei jede Raststufe (122) einen in Annäherung zum Kolben (103) hin abnehmenden Querschnitt besitzt, wobei die Rohrwand (102) des Reservoirs der Spritze mindestens einen elastischen Zahn (123) aufweist, welcher dazu ausgebildet ist, zwischen zwei aufeinanderfolgende Raststufen (122) der Kolbenstange (104) einzugreifen, und mit diesen Raststufen (122) zusammenwirkt, und wobei das mit dem Kolben verbundene Stück (106) der Spritze mindestens einen elastischen Zahn (124) aufweist, welcher dazu ausgebildet ist, zwischen zwei aufeinanderfolgende Raststufen (122) der Kolbenstange (104) einzugreifen, und mit diesen Raststufen (122) zusammenwirkt,
**dadurch gekennzeichnet**, daß das mit der Kolbenstange (104) verbundene Stück (106) die Blockierung des Kolbens gegen Längsbewegung relativ zu dem Rohrkörper durch Mittel erlaubt, welche mindestens einen Anschlag an dem Rohrkörper umfassen, an dem ein elastisch in eine Ruhestellung rückgestelltes, bewegliches Element (130) des mit der Kolbenstange (104) verbundenen Stücks (106) zur Anlage gelangt, und daß mindestens ein Teil (129) des längs der Außenwand des Reservoirs verschiebbaren Ringelements, an dem die zweite Feder (110) abgestützt ist, durch ein Fenster (132) hindurch nach außen aus dem Rohrkörper (101) der Spritze vorsteht, welcher Teil es erlaubt, die entspannte zweite Feder (110) manuell in ihre komprimierte und blockierte Anfangsstellung zu bringen.

7. Automatikspritze nach Anspruch 6,
**dadurch gekennzeichnet**, daß die Mittel zum Blockieren des mit der Kolbenstange (104) verbundenen Stücks (106) mindestens eine erste Zunge (130) umfassen, welche das mit der Kolbenstange verbundene Element bildet und mit ihrem auf der gegenüberliegenden Seite der Nadel liegenden Rand mit der Kolbenstange verbunden ist und derart von innen nach außen geneigt ist, daß ihr auf der Seite der Nadel liegender Rand in der Ruhestellung zur Anlage an einem mit dem Rohrkörper verbundenen Anschlag gelangt, und daß mindestens eine aus dem Rohrkörper (101) ausgeschnittene und mit ihrem auf der gegenüberliegenden Seite der Nadel liegenden Rand mit dem Rohrkörper (101) verbundene zweite Zunge (127) einer ersten Zunge (130) des mit der Kolbenstange (104) verbundenen Stücks derart gegenüberliegt, daß die erste Zunge (130) durch Drücken auf die zweite Zunge (127) des Rohrkörpers (101) von ihrem Stützanschlag befreit werden kann.

8. Automatikspritze nach einem der Ansprüche 6 und 7,
**dadurch gekennzeichnet**, daß das mit der Kolbenstange (104) verbundene Stück (106) mindestens eine Nase (125) aufweist, welche in einen in der Rohrwand des Reservoirs ausgebildeten axialen Schlitz (126) ragt, dessen Länge mindestens gleich der Länge der Nase (125) ist, vergrößert um den Abstand, der zwei Raststufen (122) der Kolbenstange (104) trennt.

9. Automatikspritze nach einem der Ansprüche 6 bis 8,
**dadurch gekennzeichnet**, daß am Ende der Injektion einer Dosis die das mit der Kolbenstange verbundene Stück bildenden Zungen (130) zur Anlage an demjenigen Ende des Ringelements gelangen, das dem der Abstützung der zweiten Feder dienenden gegenüberliegt, und die Blockierzungen (116) des Ringelements (108) auseinanderspreizen.
